(19) Europäisches Patentamt
European Patent Office
Office européen des brevets

(11) Veröffentlichungsnummer: **0 035 635**
**B2**

(12)
# NEUE EUROPÄISCHE PATENTSCHRIFT

(45) Veröffentlichungstag der neuen Patentschrift: **06.04.88**

(21) Anmeldenummer: **81100531.3**

(22) Anmeldetag: **24.01.81**

(51) Int. Cl.⁴: **C 07 C 46/02,** C 07 C 50/02, C 07 C 46/08, C 07 C 37/07, C 07 C 39/07

(54) **Verfahren zur Herstellung von Trimethylbenzochinon.**

(30) Priorität: **11.03.80 CH 1902/80**
**19.08.80 CH 6256/80**

(43) Veröffentlichungstag der Anmeldung:
**16.09.81 Patentblatt 81/37**

(45) Bekanntmachung des Hinweises auf die Patenterteilung:
**19.10.83 Patentblatt 83/42**

(45) Bekanntmachung des Hinweises auf die Entscheidung über den Einspruch:
**06.04.88 Patentblatt 88/14**

(84) Benannte Vertragsstaaten:
**AT BE CH DE FR GB IT LI NL**

(56) Entgegenhaltungen:
**DE-B-1 793 037**
**FR-A-2 138 030**
**US-A-3 859 365**

**JOURNAL OF ORGANIC CHEMISTRY, Band 37, Nr. 14, 1972, P.A. WEHRLI et al.: "Synthesis of trimethylhydroquinone from aliphatic precursors", Seiten 2340-3**
**BULLETIN OF THE CHEMICAL SOCIETY OF JAPAN, Band 41, 1968, T. ICHIKAWA et al.: "Synthetic studies of alfa-tocopherol. II Synthesis of trimethylbenzoquinone", Seiten 1228-1232**

(73) Patentinhaber: **F. HOFFMANN- LA ROCHE & CO. Aktiengesellschaft, CH- 4002 Basel (CH)**

(72) Erfinder: **Bartoldus, Dieter, Schützenrainweg 7, CH- 4125 Riehen (CH)**
Erfinder: **Lohri, Bruno, Dr., Schwarzackerstrasse 53, CH- 4303 Kaiseraugst (CH)**

(74) Vertreter: **Cottong, Norbert A., Grenzacherstrasse 124 Postfach 3255, CH- 4002 Basel (CH)**

**EP 0 035 635 B2**

LIBER, STOCKHOLM 1988

**Beschreibung**

Die vorliegende Erfindung betrifft ein neues Verfahren zur Herstellung von 2,3,5-Trimethylbenzochinon der Formel

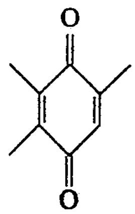

(I)

das als Zwischenprodukt in der Synthese von Vitamin E verwendbar ist.

Obwohl bereits Verfahren zur Herstellung von 2,3,5-Trimethylbenzochinon bekannt sind, besteht weiterhin ein Bedürfnis, diese Verbindung auch noch aus möglichst anderen Ausgangsstoffen und auf möglichst einfachere Art und Weise herzustellen.

Es ist z. B. bekannt, 2,5,6-Trimethyl-2-cyclohexen-1-on der Formel

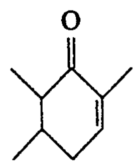

(II)

durch Dehydrieren in 2,3,6-Trimethylphenol überzuführen und dieses zu 2,3,5-Trimethylbenzochinon zu oxidieren (vgl. zum Beispiel DE-AS 1 793 037 bzw. DE-OS 2 221 624). Ein derartiges Verfahren ist auch aus J. Org. Chem. 37/14, 1972, S. 2340 ff., bekannt, wobei die Dehydrierung durch Erhitzen mit Palladium/Kohle erfolgt und die Oxidation elektrolytisch erfolgt.

Im Rahmen der vorliegenden Erfindung wurde nun gefunden, daß die vorerwähnte Dehydrierung und Oxidation in demselben organischen Lösungsmittel und mit demselben Katalysator, in einem Eintopfverfahren, durchgeführt werden können.

Die vorliegende Erfindung betrifft demnach ein Verfahren zur Herstellung von 2,3,5-Trimethylbenzochinon durch Dehydrieren von 2,5,6-Trimethyl-2-cyclohexen-1-on in einem inerten organischen Lösungsmittel und anschließende Oxidation des erhaltenen Produktes in einem inerten organischen Lösungsmittel, welches dadurch gekennzeichnet ist, daß in einem Eintopfverfahren in beiden Stufen dasselbe Lösungsmittel verwendet wird und daß beide Stufen in Gegenwart eines Kupfer(II)halogenid-Katalysators durchgeführt werden.

Beim erfindungsgemäßen Verfahren werden als inerte organische Lösungsmittel vornehmlich polare, in Wasser lösliche oder unbeschränkt mit Wasser mischbare Lösungsmittel verwendet, insbesondere solche, die sich gegenüber den Ausgangsverbindungen und den Endprodukten indifferent verhalten und in denen der Reaktionspartner und der Katalysator von Anfang bis Ende der Reaktion weitgehend in Lösung bleiben. Bevorzugt wird als inertes organisches Lösungsmittel ein Glycol, z. B. Diäthylenglycol oder Triäthylenglycol, oder ein Glycoläther verwendet. Besonders bevorzugte Lösungsmittel sind Monoglycoläther, wie Diäthylenglycolmonomethyläther oder Äthylenglycolmonomethyläther. Weiterhin sind bevorzugte Lösungsmittel Dimethylformamid und Acetonitril.

Als Kupfer(II)halogenid wird bevorzugt Kupfer(II)chlorid oder Kupfer(II)bromid verwendet.

Die in der ersten Stufe des erfindungsgemäßen Verfahrens durchgeführte Dehydrierung erfolgt vorzugsweise bei einer Temperatur zwischen 80° und 140° C, insbesondere zwischen 110° und 120° C.

Die Dehydrierung kann sowohl in Anwesenheit von Sauerstoff bzw. Luft als auch unter Inertgasatmosphäre durchgeführt werden. Nach einem bevorzugten Aspekt wird während der Dehydrierung Luft oder Sauerstoff durch das Reaktionsgemisch geleitet.

Die Oxidation gemäß Stufe 2 erfolgt vorzugsweise mit Luft oder Sauerstoff und bei einer Temperatur zwischen 60° und 120° C, insbesondere zwischen 70° und 80° C.

Sofern man die Dehydrierung unter Inertgas durchführt, wird das Kupfer(II)halogenid vorzugsweise in einem Überschuß von 4-5 Mol/Mol 2,5,6-Trimethyl-2-cyclohexen-1-on verwendet. Wird die Dehydrierung jedoch unter Luft oder Sauerstoff durchgeführt, genügen wegen der Rückoxidation von Kupfer (I) zu Kupfer (II) geringere Mengen an Katalysator. Bei der Dehydrierung unter Sauerstoff wird vorzugsweise ein Überschuß von 2 Mol Kupfer(II)halogenid/Mol 2,5,6-Trimethyl-2-cyclohexen-1-on verwendet.

Bei der anschließenden Oxidation wird praktisch alles Kupfer (I) zu Kupfer (II) rückoxidiert und kann wieder verwendet werden.

Beim Arbeiten unter Sauerstoff ist es erfindungsgemäß auch möglich, die Umsetzung des Ausgangsmaterials zum 2,3,5-Trimethylbenzo-chinon in einem Schritt durchzuführen, und zwar vorzugsweise bei einer Temperatur zwischen etwa 110- 20° C. Hierbei kann zweckmäßigerweise pro Mol Ausgangsmaterial 1 Mol Kupfer (II)halogenid, insbesondere Kupfer(II)bromid, verwendet werden.

Die nachfolgenden Beispiele erläutern die Erfindung.

**Beispiel 1**

In einem Rührkessel werden 198 g (1,16 Mol) Kupfer(II)chlorid-dihydrat in 402 g Diäthylenglycolmonomethyläther gelöst und

unter Stickstoffbegasung auf 120° C aufgeheizt. Nach Erreichen der Temperatur werden unter weiterem Einleiten von Stickstoff 42,9 g (0,31 Mol) 2,5,6-Trimethyl-2-cyclohexen-1-on innerhalb von 5 - 30 Minuten zur Lösung gegeben. Die Reaktionstemperatur steigt auf 122° C und sinkt, wenn die Gasentwicklung vorüber ist, wieder auf 116-118° C ab. Es fällt ein Niederschlag von Kupfer(I)chlorid aus. Während der Nachreaktionszeit von 20 bis 40 Minuten wird der Kolbeninhalt bei 120° C gehalten, wobei immer Stickstoff eingeleitet wird. Anschließend kühlt man das Reaktionsgemisch auf 70° C und verdrängt den vorhandenen Stickstoff durch Sauerstoff. Der Rührkessel wird verschlossen, und es wird Sauerstoff bis zu einem Überdruck von 70 bis 80 mbar zugegeben. Während der Oxidation wird der Sauerstoffdruck konstant gehalten. Das Reaktionsgemisch wird während 1,5 bis 2,5 Stunden bei 70° bis 75° C gerührt. Nach Abkühlen des Oxidationsgemisches auf 20° bis 25° C und Ablassen des Sauerstoffüberdruckes entleert man den Inhalt des Kessels in einen Scheidetrichtar. Zum Lösen der Kupfersalzniederschläge werden 40 g konzentrierte Salzsäure und 150 ml Wasser zugegeben. Das gebildete 2,3,5-Trimethylbenzochinon wird viermal mit je 250 ml Toluol extrahiert. Zum Entfernen der Kupfersalze wird die Toluolphase zweimal mit 100 ml Wasser gewaschen. Die Toluolphase wird am Rotationsverdampfer eingeengt, bis der Rückstand Toluol-frei ist. Durch eine Feinvakuumdestillation (Kp. 51 - 53° C, <0,1 mbar) wird das rohe 2,3,5-Trimethylbenzochinon von den hochsiedenden Verunreinigungen abgetrennt. Man erhält 47 - 50 g Destillat. Unter Berücksichtigung des Gehaltes an 2,3,5-Trimethylbenzochinon ergibt sich eine Ausbeute von 75 - 85 %.

**Beispiel 2**

In einem 250-ml-Vierhalskolben mit Rührer, Thermometer, Gaseinleitungsrohr und Rückflußkühler wird eine Lösung von 58,8 g (0,35 Mol) Kupfer(II)chlorid-dihydrat in 118 g Diäthylenglycolmonomethyläther vorgelegt und auf 120° C erwärmt. Nach Erreichen der Reaktionstemperatur werden 20 Liter/Stunde Sauerstoff durch die Lösung geleitet. Unter starkem Rühren werden anschließend 21,9 g (0,16 Mol) 2,5,6-Trimethyl-2-cyclohexen-1-on während 5 bis 20 Minuten zugetropft. Um die Aromatisierung zu vervollständigen, wird während 20 bis 40 Minuten bei einer Temperatur von 120° C nachgerührt. Anschließend kühlt man die Lösung auf 70° bis 75° C und oxidiert durch weiteres Sauerstoffeinleiten. Bis zum vollständigen Umsatz ist eine Reaktionszeit von 2 bis 3 Stunden erforderlich. Nach Abkühlen des Oxidationsgemisches auf 20° bis 25° C entleert man den Inhalt des Oxidationskolbens in einen Scheidetrichter und gibt 10 bis 15 ml konzentrierte Salzsäure und 100 ml Wasser zu. Anschließend extrahiert man mit einmal 200 ml und zweimal 100 ml Toluol. Die Toluolphase wird mit zweimal 50 ml Wasser gewaschen. Die Toluolphase wird am Rotationsverdampfer eingeengt, bis der Rückstand Toluolfrei ist. Der Toluol-freie Rückstand wird im Vakuum destilliert (Kp. 68° C bei 0,1 mbar). Man erhält so 18 - 20 g eines 2,3,5-Trimethylbenzochinondestillats. Unter Berücksichtigung des Gehaltes ergibt sich eine Ausbeute von 75-80 %.

**Beispiel 3**

In einem Vierhalskolben mit Rührer, Thermometer, Gaseinleitungsrohr und Rockflußkühler werden 120 g (0,54 Mol) Kupfer(II)bromid und 250 ml Dimethylformamid vorgelegt und auf 115° C erwärmt. Nach Erreichen der Temperatur werden 75,0 g (0,54 Mol) 2,5,6-Trimethyl-2-cyclohexen -1-on auf einmal zugegeben. Anschließend wird unverzüglich Sauerstoff eingeleitet. Die zu Beginn der Reaktion auftretende Exothermie wird durch vorübergehende Kühlung mit einem Kaltwasserbad kompensiert. Nach 3 Stunden Reaktionszeit bei 115° C wird die Reaktionslösung abgekühlt, mit 40 ml Wasser versetzt und mit einmal 250 ml und mit fünfmal 100 ml Toluol extrahiert. Die Toluolphasen werden vereinigt, filtriert, mit fünfmal 250 ml Wasser gewaschen und am Wasserstrahlvakuum eingedampft. Durch Vakuumdestillation (Kp. 72 - 74° C, 0,15 mbar) des Rückstandes werden 33,8 g (41 %) 2,3,5-Trimethylbenzochinon gewonnen. (Die Reaktionsbedingungen sind nicht optimiert.)

**Beispiel 4**

In einem Rührkessel werden 250 g (1,47 Mol) Kupfer(II)chlorid-dihydrat in 450 g Diäthylenglycolmonomethyläther gelöst und auf 115-117° C aufgeheizt.

Unter Durchleiten von 100 Liter/Stunde Luft durch die Katalysatorlösung werden innerhalb 5 Minuten 81 g (0,55 Mol) 2,5,6-Trimethyl-2-cyclohexen-1-on (93 %) zugegeben. Die Reaktionstemperatur steigt auf 121 - 122° C. Diese Exothermie ist von einer Gasentwicklung begleitet. Die Nachreaktionszeit beträgt total 30 Minuten. Anschließend wird die Reaktionslösung innerhalb 15 Minuten, unter weiterem Durchleiten von Luft, auf 80° C abgekühlt. Für die Oxidation verschließt man den Kessel und verdrängt die Luft durch Sauerstoff. Unter kräftigem Rühren und konstantem Sauerstoffdruck von 70 - 80 mbar beträgt die erforderliche Oxidationszeit 2 Stunden bei einer Temperatur von 80° C. Dabei werden total 15 - 17 Liter Sauerstoff verbraucht.

Das erhaltene Oxidationsgemisch wird nach

dem Abkühlen auf 20 - 25°C mit 60 g konzentrierter Salzsäure und 300 ml Wasser versetzt. Durch Extraktion mit einmal 300 ml und viermal 200 ml Methyl-tert.-butyläther trennt man das gebildete 2,3,5-Trimethylbenzochinon von der Katalysatorlösung. Die erhaltenen Lösungsmittelextrakte werden zum Entfernen restlicher Kupferchloridspuren mit fünfmal 80 ml Wasser gegengewaschen.

Die Methyl-tert.-butyläther-Phase wird anschließend am Rotationsverdampfer eingeengt und der erhaltene Rückstand dann einer Feinvakuumdestillation (50°C, <0,1 mbar) unterworfen. Man erhält ca. 75 g Destillat; unter Berücksichtigung des Gehaltes an 2,3,5-Trimethylbenzochinon ergibt sich eine Ausbeute von 78 - 80 %, bezogen auf eingesetztes 2,5,6-Trimethylcyclohexenon.

**Beispiel 5**

In einem Reaktionskessel wird die Katalysatorlösung, bestehend aus 250 g Kupfer(II)chloriddihydrat gelöst in 500 g Acetonitril, vorgelegt. Nach Aufheizen auf 78°C und unter Durchleiten von 20 Liter/Stunde Sauerstoff werden innerhalb 5 Minuten 81 g (0,55 Mol) 2,5,6-Trimethyl-2-cyclohexenon-1-on (93 %) zudosiert. Die Nachreaktionszeit muß bei konstanter Sauerstoffeinleitung und 75 - 78°C mindestens 40 Stunden betragen. Zur Aufarbeitung wird die Reaktionslösung auf 20 - 25°C abgekühlt und nach Zugabe von 50 ml konzentrierter Salzsäure und 300 ml Wasser mit dreimal 200 ml Toluol extrahiert. Die erhaltenen Toluolextrakte werden mit zweimal 100 ml Wasser gegengewaschen und am Rotationsverdampfer eingeengt. Nach einer Feinvakuumdestillation (50°C, <0,1 mbar) erhält man 82 g Destillat mit einem Gehalt an 2,3,5-Trimethylbenzochinon von 55 %, entsprechend einer Ausbeute von ca. 55 %, bezogen auf eingesetztes 2,5,6-Trimethylcyclohexenon (nicht optimiert).

**Patentansprüche**

1. Verfahren zur Herstellung von 2,3,5-Trimethylbenzochinon der Formel

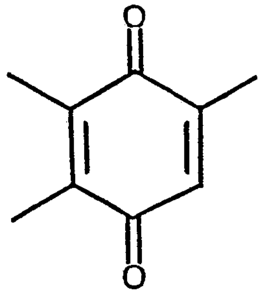

I

durch Dehydrieren von 2,5,6-Trimethyl-2-cyclohexen-1-on der Formel

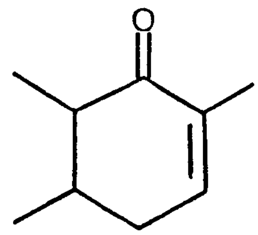

II

in einem inerten organischen Lösungsmittel und anschliessende Oxidation des erhaltenen Produktes in einem inerten organischen Lösungsmittel, dadurch gekennzeichnet, dass in einem Eintopfverfahren in beiden Stufen dasselbe Lösungsmittel verwendet wird, und beide Stufen in Gegenwart eines Kupfer(II)halogenid-Katalysators durchgeführt werden.

2. Verfahren nach Anspruch 1, dadurch gekennzeichnet, daß man als inertes organisches Lösungsmittel ein Glycol, einen Glycoläther oder Dimethylformamid verwendet.

3. Verfahren nach Anspruch 2, dadurch gekennzeichnet, daß man als Glycoläther einen Monoglycoläther, wie Diäthylenglycolmonomethyläther oder Athylenglycolmonomethyläther, verwendet.

4. Verfahren nach einem der Ansprüche 1 - 3, dadurch gekennzeichnet, daß man als Katalysator Kupfer(II)chlorid oder Kupfer(II)bromid verwendet.

5. Verfahren nach einem der Ansprüche 1 - 4, dadurch gekennzeichnet, daß die Dehydrierung bei einer Temperatur von 80° bis 140°C, vorzugsweise 100° bis 120°C, durchgeführt wird.

6. Verfahren nach einem der Ansprüche 1 - 5, dadurch gekennzeichnet, daß die Dehydrierung unter Stickstoff durchgeführt wird.

7. Verfahren nach einem der Ansprüche 1 - 5, dadurch gekennzeichnet, daß die Dehydrierung unter Sauerstoff oder Luft durchgeführt wird, wobei Luft oder Sauerstoff vorzugsweise durch das Reaktionsgemisch geleitet wird.

8. Verfahren nach einem der Ansprüche 1 - 7, dadurch gekennzeichnet, daß man die Oxidation mit Luft oder Sauerstoff durchführt.

9. Verfahren nach einem der Ansprüche 1 - 8, dadurch gekennzeichnet, daß man die Oxidation bei einer Temperatur von 50° bis 120°C, vorzugsweise bei 70° bis 80°C, durchführt.

10. Verfahren nach Anspruch 1 oder 2, dadurch gekennzeichnet, daß man als Lösungsmittel Dimethylformamid und als Katalysator Kupfer(II)bromid verwendet, daß die Umsetzung von 2,5,6-Trimethyl-2-cyclohexen-1-on zum 2,3,5-Trimethylbenzochinon in einem Schritt unter Sauerstoff bei einer Temperatur von etwa 110 - 120°C durchgeführt wird, wobei pro Mol 2,5,6-Trime-thyl-2-cyclohexen-1-on 1 Mol Kupfer(II)bromid verwendet wird.

**Claims**

1. Process for the manufacture of 2,3,5-trimethylbenzoquinone of the formula

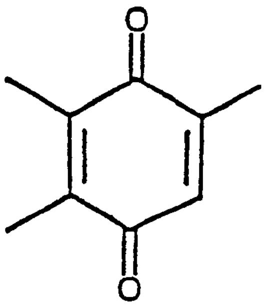

I

by dehydrogenating 2,5,6-trimethyl-2-cyclohexen-1-one of the formula

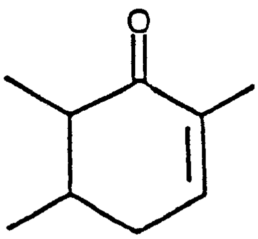

II

in an inert organic solvent and subsequently oxidizing the resulting product in an inert organic solvent, characterized by using in a one-pot process the same solvent in both steps and by carrying out both steps in the presence of a copper (II) halide catalyst.

2. Process according to claim 1, characterized in that a glycol, a glycol ether or dimethylformamide is used as the inert organic solvent.

3. Process according to claim 2, characterized in that a monoglycol ether, such as diethylene glycol monomethyl ether or ethylene glycol monomethyl ether, is used as the glycol ether.

4. Process according to any one of claims 1 - 3, characterized in that copper (II) chloride or copper (II) bromide is used as the catalyst.

5. Process according to any one of claims 1 - 4, characterized in that the dehydrogenation is carried out at a temperature of 80° to 140° C, preferably 100° to 120° C.

6. Process according to any one of claims 1 - 5, characterized in that the dehydrogenation is carried out under nitrogen.

7. Process according to any one of claims 1 - 5, characterized in that the dehydrogenation is carried out under oxygen or air, whereby air or oxygen is preferably conducted through the reaction mixture.

8. Process according to any one of claims 1 - 7, characterized in that the oxidation is carried out with air or oxygen.

9. Process according to any one of claims 1 - 8, characterized in that the oxidation is carried out at a temperature of 50° to 120° C, preferably at 70° to 80° C.

10. Process according to claim 1 or 2, characterized in that dimethylformamide is used as the solvent and copper (II) bromide is used as the catalyst, in that the conversion of 2,5,6-trimethyl-2-cyclohexen-1-one into 2,3,5-trimethylbenzoquinone is carried out in one step under oxygen at a temperature of about 110 - 120° C, whereby 1 mol of copper (II) bromide is used per mol of 2,5,6-trimethyl-2-cyclohexen-1-one.

**Revendications**

1. Procédé de préparation de la 2,3,5-triméthylbenzoquinone de formule

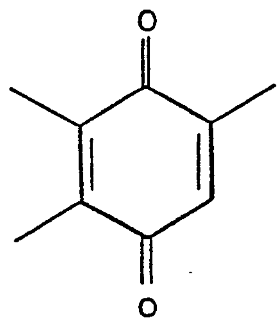

I

par déshydrogénation de la 2,5,6-triméthyl-2-cyclohexène-1-one de formule

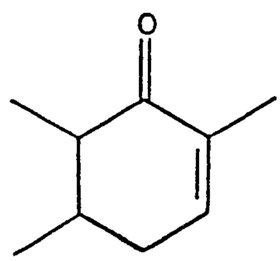

II

dans un solvant organique inerte avec oxydation subséquente du produit obtenu dans un solvant organique inerte, caractérisé en ce que l'on utilise, en un seul et même récipient, le même solvant dans les deux stades opératoires et en ce que l'on exécute les deux stades opératoires en présence d'un catalyseur consistant en un halogénure de cuivre-II.

2. Procédé selon la revendication 1, caractérisé en ce que l'on utilise en tant que solvant organique inerte un glycol, un éther de glycol ou le diméthylformamide.

3. Procédé selon la revendication 2, caractérisé en ce que l'on utilise en tant qu'éther de glycol un monoéther de glycol comme l'éther monométhylique du diéthylène-glycol ou l'éther monométhylique de l'éthylène-glycol.

4. Un procédé selon l'une des revendications 1 à 3, caractérisé en ce que l'on utilise en tant que catalyseur le chlorure de cuivre-II ou le bromure de cuivre-II.

5. Procédé selon l'un des revendications 1 à 4, caractérisé en ce que l'on procéde à la

déshydrogériation à une température de 80 à 140° C, de préférence de 100 à 120° C.

6. Procédé selon l'une des revendications 1 à 5, caractérisé en ce que la déshydrogénation est effectuée en atmosphère d'azote.

7. Procédé selon l'une des revendications 1 à 5, caractérisé en ce que la déshydrogénation est effectuée en atmosphère d'oxygène ou d'air, l'air ou l'oxygène étant de préférence injecté dans le mélange de réaction.

8. Procédé selon l'une des revendications 1 à 7, caractérisé en ce que l'on procéde à l'oxydation à l'aide d'air ou d'oxygène.

9. Procédé selon l'une des revendications 1 à 8, caractérisé en ce que l'oxydation est effectuée à une température de 60 à 120° C, de préférence de 70 à 80° C.

10. Procédé selon la revendication 1 ou 2, caractérisé en ce que l'on utilise en tant que solvant le diméthylformamide et en tant que catalyseur le bromure de cuivre-II, en ce que l'on convertit la 2,5,6-triméthyl-2-cyclohexène-1-one en la 2,3,5-triméthyl-benzoquinone en une étape en atmosphère d'oxygène à une température d'environ 110 à 120° C en utilisant 1 mole de bromure de cuivre-II par mole de 2,5,6-triméthyl-2-cyclohexène-1-one.